# EUROPÄISCHE PATENTSCHRIFT

(11) **EP 2 833 842 B1**
(45) Veröffentlichungstag und Bekanntmachung des Hinweises auf die Patenterteilung: **31.01.2018**
(21) Anmeldenummer: 13721548.9
(22) Anmeldetag: 03.04.2013
(51) Int. Cl.: A61F 2/44, A61F 2/46, A61F 2/30

(54) **WIRBELSÄULEN-IMPLANTAT, WERKZEUGE ZUM EINBRINGEN VON IMPLANTATEN ZWISCHEN ZWEI WIRBELKÖRPER EINER WIRBELSÄULE UND OPERATIONSSET**
SPINAL IMPLANT, TOOLS FOR INTRODUCING IMPLANTS BETWEEN TWO VERTEBRAL BODIES OF A SPINAL COLUMN AND SURGICAL KIT
IMPLANT DE COLONNE VERTÉBRALE, OUTILS PERMETTANT D'INTRODUIRE DES IMPLANTS ENTRE DEUX CORPS VERTÉBRAUX D'UNE COLONNE VERTÉBRALE ET KIT DE BLOC OPÉRATOIRE

(30) Priorität: 05.04.2012 DE 102012007032
(43) Veröffentlichungstag der Anmeldung: 11.02.2015
(73) Patentinhaber: Spine Center Baden GmbH, 77654 Offenburg (DE)
(72) Erfinder: HOELL, Thomas, 77815 Buehl (DE); BEIER, Andre, 77815 Buehl (DE)
(74) Vertreter: Ege Lee & Partner Patentanwälte PartGmbB
(86) Internationale Anmeldenummer: PCT/DE2013/000177
(87) Internationale Veröffentlichungsnummer: WO 2013/149611

(56) Entgegenhaltungen:
- WO-A1-2005/087143
- WO-A2-2011/013047
- FR-A1- 2 923 158
- US-A1- 2002 019 637
- US-A1- 2011 264 218
- US-A1- 2011 301 710
- US-B1- 7 905 886

## Beschreibung

Die Erfindung bezieht sich auf Wirbelsäulen-Implantate, auch Cages genannt, sowie Werkzeuge, Geräte bzw. Instrumente und Operations-Sets zum Einbringen von insbesondere solchen Implantaten zwischen zwei benachbarte Wirbel einer Wirbelsäule.
Zum Einführen von Implantaten in den Zwischenraum zwischen zwei benachbarte Wirbelkörper einer Wirbelsäule haben sich in der Humanmedizin im wesentlichen zwei unterschiedliche Formen von Implantaten herausgebildet, nämlich einerseits gebogene, auch boomerangförmige oder Banana-Cage genannte und andererseits zumindest annähernd symmetrische gerade Cages, die jeweils mittels spezieller Werkzeuge und Operationstechniken implantiert werden. In beiden Fällen werden die im Zentrum hohl ausgebildeten Cages zuvor mit Knochensplittern aus dem Zugangsbereich oder synthetischem Knochenersatzmaterial ausgefüllt. Nach dem Einsetzen des Cages werden in den meisten Fällen die beteiligten Wirbel über ein Schraubensystem versteift.
Banana- Cages werden von dorsal einseitig in den Raum zwischen zwei Wirbeln eingebracht und dort in der Transversalebene verdreht bzw. verschwenkt. Dabei soll sich der konvexe Bereich des Cage entlang des konkaven Bereiches der Ringapophyse anlegen.
Für das Einbringen und Verschwenken bzw. Rotieren solcher Cages gibt es im Grunde drei unterschiedliche Gruppen von Werkzeugen, wovon zunächst diejenige Gruppe genannt sei, bei der der Cage zwangsweise, gewissermaßen von außen ferngesteuert, verdreht bzw. verschwenkt werden soll. Hierunter fallen solche Werkzeuge bzw. Methoden, bei denen der Cage, nachdem er in den Raum zwischen zwei Wirbel verbracht wurde, über Drahtzüge verschwenkt wird, die in dem Werkzeug, das noch weitere Funktionen übernehmen muß, geführt sind. Zunächst soll jedoch zum Einbringen des Implantates in den Zwischenwirbelraum der Cage möglichst starr mit dem Werkzeug verbunden werden. Dieses weist an seinem distalen Ende das kugelige Bauteil des Gelenkes auf und dieses einen Gewindefortsatz, auf den der Cage aufgeschraubt wird. Das kugelartige Bauteil kann mitsamt dem Cage in verschiedene Lagen gegenüber dem Werkzeug gebracht werden. Über einen Drehmechanismus, der am proximalen Ende des Werkzeuges vorgesehen ist und mit einer Gewindestange zum Kugelgelenk hin, kann das Kugelgelenkteil gegen das Ende des rohrförmig ausgebildeten Werkzeuges gezogen werden. Dadurch soll der Cage in eine vorbestimmte Lage gegenüber dem Werkzeug festgelegt werden, um so in den Zwischenraum zwischen zwei Wirbel eingebracht zu werden. Nach dem Einbringen kann durch Lösen der Verklemmung zwischen kugelartigem Gelenkteil und Stabende die Verspannung zwischen Kugelgelenkteil und Inserterstab gelockert werden und durch entsprechende Betätigung des Drahtzuges, der um den Cage gelegt ist, soll der Cage weiter verschwenkt werden. Solche Werkzeuge bzw. Cages und Methoden sind beispielsweise durch die FR 29 23 158 bekannt geworden.

Es hat sich bei dieser Ausführungsform jedoch gezeigt, dass die Gelenke und somit die Cages am Werkzeug nicht so fest und starr adaptiert werden können, dass sie den beim Eintreiben zwischen den Gelenkteilen auftretenden hohen Belastungen standhalten. Die durch Schläge auf den Griff ausgeübte Krafteinwirkung beansprucht diese Gelenke massiv, so dass beim Eintreiben die Gefahr einer Knickbildung zwischen Werkzeug und Cage auftritt. Der Stab weicht dabei nach lateral aus und kann den Wirbelknochen und die nahe der Insertionsstelle liegende Nervenwurzel und/oder andere Organe beschädigen. Die WO 2011/0013047 zeigt eine solche Gestaltung.

Ein weiteres Beispiel für ein von außen gesteuertes Verdrehen des Cages im Raum zwischen den beiden Wirbeln zeigt die EP 1 596 764. Dabei ist am distalen Ende des Werkzeuges ein Mechanismus angeordnet, über den zunächst der Cage zum Eintreiben in den Zwischenraum starr mit dem Stab verbunden werden soll, damit er beim Einschlagen nicht auskippt.

Derselbe Mechanismus dient sowohl für das Eintreiben, als auch weiterhin für das gesteuerte Verschwenken des Cage, sobald sich dieser in dem Raum befindet. Am Ende des Werkzeuges ist außerdem noch eine Spreizeinrichtung vorgesehen, um den Cage zwischen den zwei Wirbeln aufzuweiten. Darüber hinaus ist auf dem Ende des Stabes auch noch ein Ausklinkmechanismus vorgesehen, der den Stab vom Cage trennen soll, sobald letzterer in der gewünschten Lage ist. Es hat sich jedoch gezeigt, dass Anordnungen, bei denen versucht wird, eine Vielzahl von Funktionen in das Stabende bzw. die Übergangsmechanik zwischen dem Werkzeug und dem Cage hinein zu verlegen, nämlich einerseits eine starre Verbindung zwischen Werkzeug und Cage für das Eintreiben zu schaffen und andererseits den Verschwenkmechanismus und hier auch noch den Spreizmechanismus und dazu noch den Ausklinkmechanismus, bei den vorherrschenden geringen Platzverhältnissen entweder die eine oder die andere oder gar alle Funktionen nicht mit hinreichen Sicherheit verwirklicht werden können. Es besteht also auch hier die Gefahr des Ausknickens des Werkzeuges. Im Zusammenhang auf weitere Ausgestaltungen, bei denen Arretier- und Verschwenkeinrichtung zusammengelegt sind, sei beispielhaft auch verwiesen auf eine erste der in der US 2006/0235426 gezeigten Ausführungsformen sowie auf die US 2008/0140085 und die US 2008/0091211.

Eine weitere Gruppe von Werkzeugen und Cages, bei der der Cage nach dem Einbringen innerhalb des Bandscheibenzwischenraums verschwenkt werden soll, ist in der WO 2010/121030 offenbart. Hier ist ebenfalls am distalen Ende eines hohlen stabförmigen Werkzeuges eine dort festklemmbare und lösbare Gelenkverbindung vorgesehen und an dem gegenüber dem Stab verschwenkbaren Gelenkteil ist ein Gewindestift angeordnet, auf dem der Cage befestigbar ist. Durch Betätigen des am proximalen Ende des Gerätes befindlichen Handgriffs kann die Winkelposition des Cage gegenüber dem Werkzeug verändert werden. Hier wird ebenfalls versucht, zum Zwecke des Eintreibens des Cage in den Zwischenwirbelraum, die gelenkartige Verbindung zu stabilisieren. Nachdem der Cage in den Zwischenraum eingebracht worden ist, wird versucht, diesen zu verschwenken. Hierfür wird das gesamte Werkzeug, soweit es die für diesen Zweck eingebrachte Wunde erlaubt, in der Transversalebene verschwenkt. Danach wird durch Drehen des Griffes die Verspannung im Gelenk gelöst, das Werkzeug auf die andere Seite der Wunde verschwenkt, dort wird versucht, durch Verdrehen des Griffes die Gelenkverbindung wieder möglichst starr zu gestalten, um durch Verschwenken des Werkzeuges in der Wunde wieder zurück, den Cage weiter zu verdrehen. Dieser Vorgang wird so oft als nötig wiederholt. Auch hier hat sich gezeigt, dass die gelenkige Verbindung, die über Reibung fixiert wird, für die hohen, beim Eintreiben auftretenden Kräfte in vielen Fällen nicht ausreicht, sodass die beim Ausknicken bestehenden Verletzungsgefahren bestehen. Offensichtlich deshalb wurde in dieser Schrift auch noch vorgesehen, innerhalb der gelenkigen Verbindung eine formschlüssige Verbindung in Form von Klinken oder Verzahnungen vorzusehen. Aber auch eine solche Ausgestaltung hält den auftretenden Belastungen nicht dauerhaft stand, weil die Verzahnungen oder der Klinkenmechanismus sowie die weitern dort vorgesehenen Funktionselemente wegen der Platzverhältnisse nur äußerst filigran ausgeführt werden können, was entweder eine frühzeitige Aussonderung des Instruments erfordert oder aber zum Ausknicken führt mit den damit verbundenen Folgen. Diese Gefahr erhöht sich bei Werkzeugen der letzterwähnten Art auch noch deshalb, weil nicht erkennbar ist, wie weit der Verschleiß bereits fortgeschritten ist und daher nicht voraussehbar ist, wann das Werkzeug versagt. Bei weiteren Ausführungsbeispielen ist außerdem noch ein Ausklinkmechanismus zum Lösen des Werkzeuges vom eingebrachten Cage im Bereich der gelenkigen Verbindung vorgesehen. Dies erfordert eine weitere Reduzierung der Dimensionen der einzelnen Elemente.

Bei einer dritten Gruppe von Werkzeugen zum Einbringen und Verschwenken eines Banana-Cage, wie es beispielsweise durch die WO 2005/041825 bekannt geworden ist, soll sich der Cage, sobald er zwischen die Wirbel eingebracht ist, von selbst in ein eine schräge Lage parallel zum ventralen Anulus einfädeln. Zunächst wird zum Eintreiben desselben zwischen diesem und dem Werkzeug - einem Hohlstab - wiederum eine reibschlüssige Verbindung hergestellt, indem der Cage mit einem abgerundeten Ende über eine im Hohlstab vorgesehene Zugstange gegen eine abgerundete Fläche des distalen Endes des Werkzeuges verspannt wird. Nach dem Einbringen des Cage soll sich dieser infolge der aufgebrachten Schlagimpulse durch Deflexion, also durch Abgleiten, im Zusammenwirken seiner konvex ausgebildeten Fläche mit der konkaven Fläche der Ringapophyse von selbst verdrehen, wofür nach dem Eintreiben die Verbindung zwischen Cage und Werkzeug zu lockern ist. Abgesehen davon, dass auch hier wiederum mehrere Funktionen in den Endbereich des Werkzeuges verlegt worden sind, hat sich gezeigt, dass die Annahme, der Cage würde eine selbsttätige Bewegung über die Mittellinie des Bandscheibenraumes hinweg und entlang der Ringapophyse ausführen und sich selbst automatisch in die anatomisch gewünschte Position bringen, im Normalfall nicht oder nur unzulänglich funktioniert. Dies ist unter anderem damit zu erklären, dass der Innenraum zwischen den Wirbelkörpern, obwohl oder gerade weil er teilweise ausgehöhlt ist, dem Cage unterschiedlichen Widerstand entgegensetzt. Typischerweise verklemmt sich die Spitze des Cages in Einschlagrichtung zwischen den Ringapophysen.

Aus den genannten Gründen ist die Verwendung von Banana-Cages rückläufig.

Die zweite genannte Form von Cages, also die zumindest im wesentlichen geraden Cages, können nach der so genannten PLIF (Posterior Lumbar Interbody Fusion) - oder nach der TLIF (Transforminal Lumbar Interbody Fusion) - Operationstechnik eingebracht werden.

Bei der PLIF- Versorgung wird der Cage von dorsal mit einem fluchtenden und fest verschraubten Inserter-Stab, ca. 10° zur Körpermitte geneigt, eingeschlagen. Der Nachteil bei dieser Methode ist, dass, um eine ausreichende Stabilisierung der Wirbel zu erzielen, zwei Cages erforderlich sind, was auch eine beidseitige Präparation des Spinalkanals, mit entsprechendem Zeitaufwand und entsprechender Risikoverdoppelung in diesem Bereich erfordert.

Nachdem eine beidseitige Versorgung insbesondere bei Fällen mit Voroperationen gegebenenfalls mühsam und gefährlich ist, wurde eine einseitige Operationstechnik in Form des TLIF entwickelt. Es hat sich seit einigen Jahren gezeigt, dass lediglich ein einziger, im Bandscheibenraum etwa diagonal platzierter Cage ausreichend für die Primärstabilität und spätere Fusion der benachbarten Wirbelkörper ist. Bei der TLIF-Versorgung, bei der der Cage in einem 30-45° Winkel im Bandscheibenraum zu liegen kommt, ist es für diese diagonale Einbringung erforderlich, das Wirbelgelenk und gegebenenfalls auch Teile des Wirbelbogens zu entfernen, um mit dem Cage und dem Werkzeug in der oben beschriebenen Richtung eintreten zu können. Der Cage wird bei dieser Methode also von weiter seitlich in den Bandscheibenraum eingebracht als bei dem PLIF-Verfahren. Es gibt weitere Verfahren, von seitlich, retroperitoneal bis ca 90° seitliche Cages einzubringen und es gibt die ventralen Zugangsverfahren, die jedoch von der vorliegenden Erfindung weiter entfernt sind als die oben beschriebenen.
Die Vorteile der TLIF-Technik sind, dass lediglich ein einziges Implantat und auch nur von einer Seite eingebracht wird. Diese Operationstechnik spart neben dem zweiten Implantat im Mittel eine halbe bis eine Stunde Operationszeit, es wird lediglich ein Cage verwendet und die Risiken einer bilateralen Nervenwurzelpräparation sind ausgeschaltet. Der Nachteil ist jedoch die weitgehende Resektion des Wirbelgelenkes auf der Zugangsseite und die erhöhte Gefahr der Nervenwurzelverletzung, da der Cage ja von einer Position näher der kranial verlaufenden Nervenwurzel eingeführt wird als beim PLIF.
Aus der WO 2005/087143 A1 ist ein Wirbelsäulen-Implantat mit einem länglichen Körper mit zumindest annähernd gerade verlaufender Achse bekannt, der an einem Endbereich eine Kontur als Angriffsfläche zur gelenkigen Verbindung mit einem anderen Werkzeug aufweist.
Aus der US 2002/019637 A1 ist ein Werkzeug zum Implantieren eines Wirbelsäulen-Implantats mit einer stabförmigen Ausbildung mit einem proximalen Endbereich mit einem als Handgriff ausgebildeten Endbereich und einem distalen Endbereich bekannt, wobei die Endbereiche achsversetzt zueinander angeordnet sind.
Der vorliegenden Erfindung lag die Aufgabe zugrunde, die mit den bisher bekannten Cages und Werkzeugen und mit den bisher angewandten Methoden beim Inserieren verbundenen Nachteile zu beseitigen und die Möglichkeit zu schaffen, das Einführen und Platzieren von Cages in den Raum zwischen zwei Wirbeln einer Wirbelsäule auf sichere, möglichst verletzungsfreie, preiswerte und zeitsparende Weise zu gewährleisten und hierfür geeignete Cages und einfache und preiswerte Werkzeuge, die praktisch nicht verschleißen, zu schaffen.
Dies wird gemäß der vorliegenden Erfindung mittels eines Cage verwirklicht, der als länglicher Körper mit zumindest annähernd gerader Achse ausgebildet ist, der auf wenigstens einem seiner Endbereiche eine Kontur als Angriffsfläche zur starren, jedoch lösbaren Verbindung mit einem ersten Werkzeug aufweist und eine weitere Kontur als Angriffsfläche zur vorübergehenden Bildung einer gelenkigen Verbindung mit einem anderen Werkzeug. Dabei sind beide Konturen innerhalb des Implantats vorgesehen. Vorteilhaft ist es weiterhin, wenn, vom Endbereich aus gesehen, die weitere Kontur, also die als Angriffsfläche für die gelenkige Verbindung dienende Kontur vor der Kontur der Angriffsfläche für die Bildung der starren Verbindung vorgesehen ist. Weiterhin ist es vorteilhaft, wenn die Kontur zur Bildung der gelenkigen Verbindung als runder Hohlkörper ausgebildet ist, z.B. kalottenartig, vorzugsweise kugel kalottenartig.
Die Kontur zur Bildung der festen bzw. starren Verbindung mit einem Werkzeug ist zweckmäßigerweise eine Gewindebohrung, wobei weiterhin die Ausbildung derart getroffen werden kann, dass die beiden Konturen ineinander übergehen. Dabei ist es vorteilhaft, wenn - vom Endbereich des Implantats aus gesehen - die kalottenartige Ausbildung zuerst kommt und danach der Innengewindebereich, die zweckmäßigerweise beide achsgleich angeordnet sein können.

Zweckmäßig kann eine solche Ausgestaltung des Cage sein, bei der die beiden Konturen lediglich an einem Ende- dem Heck - vorgesehen sind und der vordere Bereich - der Bug - bombiert sein kann.

Weiterhin kann es vorteilhaft sein, wenn wenigstens eine der den Endplatten zugewandten Oberflächen profiliert ist, was beispielsweise als Sägezahnmuster, als quer zur Achse verlaufende Rille(n), Riffelung oder als Rauheit ausgeprägt wird, deren Aufgabe es ist, jeweils die größte Reibung in der Richtung zu vermitteln, aus der der Cage eingetrieben wurde und den Cage damit gegen ein herausrutschen aus dem Bandscheibenfach zu sichern.

Ein Implantat gemäß der vorliegenden Erfindung, das an zumindest einem seiner Endbereiche eine eingebrachte kalottenartige oder zylinderföriger Hohlkörper, insbesondere eine kugelkalottenartige Einbuchtung aufweist und weiter innen eine Gewindebohrung und eine längliche Struktur mit gerader Achse hat, vereinigt in einfacher, preiswerter und sicherer Weise die Vorteile der PLIF- und der TLIF- Versorgung. So braucht lediglich ein einziger Cage pro benachbartem Wirbelpaar eingesetzt zu werden, und zwar in der der PLIF entsprechenden relativ schonenden Weise, nachdem wie üblich zuvor das Facettengelenk des entsprechenden Wirbelkörpers teilweise präpariert und Knorpelgewebe aus dem Bereich des Bandscheibenfaches entnommen wurde.

Die Fusion des Cages wird durch die Befüllung mit Knochenmaterial aus dem Zugangsbereich oder mit synthetischen Knochenersatzmaterialien erleichtert.

Zum Implantieren wird der Cage an einem Werkzeug, dem Inserterstab, befestigt. Dieser besteht aus einem Hohlstab und einem darin vorgesehenen Innenteil bzw. einer Seele mit am distalen Ende vorgesehenen Gewindebereich, auf den der erfindungsgemäße Cage aufgeschraubt wird, nachdem der Gewindebereich zunächst durch den vorzugsweise kalottenartigen Bereich des Cage hindurchgeführt wurde. Der proximale Bereich der Seele ist an einem Knopf bzw. Drehgriff des Inserterstabes festgelegt und zwischen Knopf und Hohlstab befindet sich ein Knauf mit Knebeln. Durch Verdrehen des Knopfes gegenüber dem Knebel wird der Cage fest gegen den Hohlstab verspannt. Cage und Inserter bilden damit eine starre Einheit. Der Hohlstab überträgt die Schlagkraft auf den Cage, sodass Gewinde und Seele geschont werden. Der Hohlstab ist an seinem dem Cage zugewandten Endbereich vorzugsweise der entsprechenden Endkontur des Cage bzw. dem Heck desselben zumindest annähernd angepasst. Er kann dort ggf. zusätzlich gabel- oder zinkenartig ausgebildet auch erweitert sein.

Damit ist der Cage geeignet, mit Hilfe des Werkzeuges zunächst lediglich teilweise, vorzugsweise jedoch überwiegend, vorzugsweise mit seinem hinteren Ende im Bereich des äußeren Anulus ggf. geringfügig darüber hinausragend , durch Schlagimpulse auf das proximale Ende des Werkzeuges eingebracht zu werden, und zwar in eine der PLIF zwar winkelmäßig entsprechende -also eine etwa 10°-Lage, jedoch der PLIF-Endlage noch nicht ganz entsprechende Lage.

Hierfür wird der Cage demgemäß durch Schläge auf das hintere Ende des Inserter-Werkzeuges so weit in den Bandscheibenraum eingebracht, bis sein hinteres Ende in Höhe des Anulus der Bandscheibe liegt. Der Inserter wird ausgeschraubt.

Danach kommt ein zweites spezielles Werkzeug zum Einsatz, das hier "Ball Tool" genannt sei. Dieses ist an seinem distalen Endbereich zweckmäßigerweise kugelartig ausgebildet und geeignet, in die weitere Kontur des Cage, die, wie erwähnt, kalottenförmig gestaltet sein kann, eingebracht zu werden zum weiteren Eintreiben und gleichzeitigen Verdrehen bzw. Verschwenken in eine dem TLIF zumindest angenäherte Lage. Dieses Werkzeug ist derart ausgebildet, dass es, wenn es dorsal durch die gleiche Präparation eingebracht wird wie zuvor der Inserterstab, durch Aufbringen von Schlagimpulsen auf das proximale Ende, also auf das Ende des Griffbereiches, beim weiteren Eintreiben gleichzeitig eine Verschwenkung des Cage bewirkt in eine mehr diagonale Lage, als dies nach dem PLIF-Verfahren möglich ist. Hierfür ist das Werkzeug vorteilhafterweise in seinem distalen Endbereich abgekröpft, die Achse des Griffes und des distal gelegenen Bereiches sind also versetzt. Die Richtung der Stoßimpulse, die durch Schläge auf den Endbereich des Griffes entstehen, verläuft also ebenso versetzt zum kugelartigen Ende des Werkzeuges bzw. dem Angriffsbereich im Cage. Die Abknickung im stabförmigen Bereich des Werkzeuges weist dabei weg vom Dorn des Wirbelkörpers, also nach lateral, entsprechend weg von der Körpermitte gerichtet, so dass beim Aufbringen von Schlagimpulsen Kraftkomponenten auf den Cage einwirken sowohl in Richtung nach ventral (bauchseitig) als auch nach lateral, der Cage driftet dadurch also gewissermaßen zumindest mit seinem hinteren Ende in eine zumindest annähernd diagonale Lage, also in eine zumindest annähernd dem TLIF entsprechende Winkellage. Das "Ball Tool" wird danach entfernt.

In einfachen Fällen befindet sich der Cage bereits jetzt in einer solchen diagonalen Lage, dass sie als Endposition im Bandscheibenraum ausreichend ist.

Sollte der Cage jedoch noch nicht in einer dem Operateur günstig erscheinenden Position sein, erfolgt der dritte Schritt, bei dem mit einem als "Nachschläger" zu bezeichnenden weiteren Werkzeug auf den Außenbereich des Heck des schräg stehenden Cages geschlagen wird, um die Position des Cage durch weiteres Verschwenken, Verdrehen bzw. Driften und durch weiteres Eintreiben nach ventral, noch zu korrigieren. Dieses Werkzeug kann an seinem distalen Ende gabelförmig ausgebildeten sein, wobei es vorteilhaft sein kann, wenn das Ende dieses Werkzeuges den entsprechenden Konturen des Cage zumindest teilweise angepasst ist. Als solches, weiteres Werkzeug kann aber auch der Hohlstab des Inserter-Werkzeuges verwendet werden, aus dem der Kopf mitsamt der Seele entfernt wurde.

Durch dieses Nachschlagen wird der Cage sowohl in den Bandscheibenraum eingetrieben, nach ventral, als auch noch schräger in den Raum eingebracht, idealerweise kommt das Heck auf eine ähnlich Höhe wie die Spitze, der Cage liegt dann quer im Bandscheibenraum. Wird diese quere Lage nicht erreicht und der Cage behält eine Winkel von 30-50°, ist dies nach dem aktuellen Stand der Chirurgie auch eine korrekte Lage.

Für Cages, insbesondere solchen aus Kunststoff, ist es vorteilhaft, eingebrachte Markierungselemente aus Metall oder anderem röntgendichten Material zu verwenden, die vorteilhafterweise am vorderen und hinteren Bereich, zweckmäßigerweise jeweils in zueinander versetzter Richtung, eingebracht sind, sodass mittels Röntgenstrahlung die Lage des Cage während der Operation beobachtet werden kann (Fluoroskopie, Computer Tomographie).

Durch die vorliegende Erfindung sind gegenüber den bisher bekannten Cages und den verwendeten Werkzeugen sowie den bekannten Verfahren zum Einbringen erhebliche Vorteile gegeben. So sind z.B. die Vorteile des PLIF, nämlich u. a. das Einbringen des Cage an einer chirurgisch günstigen Stelle über einen vergleichsweise kleinen dorsalen Zugang in der Haut, in einer annähernd 10 °-Position zur Mittelachse und die Vorteile des TLIF vereinigt, nämlich die Verwendung von nur einem einzigen Cage.

Die Gefahren einer Beschädigung des Cage oder sogar des Patienten, wie dies bei bananenförmigen Cages im Zusammenwirken mit den entsprechenden Werkzeugen erfolgen kann, sind praktisch ausgeschlossen. Beim Eintreiben sind Inserter und Cage nicht über eine Reibverbindung zueinander gehalten, sondern fest miteinander verschraubt und verspannt, damit ist ein Ausknicken der Verbindung zwischen Cage und Inserter ausgeschlossen. Beim Verschwenken des Cage mit dem Ball Tool ist die Kraftübertragung ebenfalls sicher, weil das Ball tool im der Kugelkallote des Cages liegt.

Die Werkzeuge weisen keine Reibverbindungen oder Gelenke auf, die verschleißen können, es kann deshalb praktisch nicht beschädigt werden. Dadurch ist die Verlässlichkeit des Werkzeuges für den Operateur auch nach vielen Operationen gegeben. Unerwartete Reaktionen wie das plötzliche Ausknicken eine reibschlüssigen Verbindungen sind vollständig ausgeschlossen.

Dies begünstigt wiederum den vermehrten Einsatz von Kunststoffen wie etwa PEEK (Poly Ether Ether Keton) für die Cages, ebenso den Einsatz von anderen Materialien und Zusammensetzungen, die wegen der obigen Nachteile bisher nicht eingesetzt werden konnten. Ebenso entfällt die aufwändige Präparation des TLIF, das Wirbelgelenk kann partiell erhalten werden. Damit ist die Möglichkeit einer dorsalen Anlagerung von Knochenmaterial gegeben. Darüber hinaus können die einzelnen Werkzeuge speziell auf ihre Funktion hin optimiert und schmaler und schlanker ausgeführt werden, was gegenüber TLIF und den Banana- Cages eine noch geringere Präparation im Bereich des Facettengelenkes erlaubt. Die hier vorgestellte schlanke Ausführung der Werkzeuge ist von praktischer Bedeutung, weil die Eintrittsfläche in den Bandscheibenraum nur maximal ca. 8 bis 10x10 mm groß ist. Kombinierte Werkzeuge sind in der Regel größer und erlauben nicht den Blick auf die Insertionsstelle respektive den Duralschlauch und die Nervenwurzeln. Des Weiteren ist die Gefahr, den kranial verlaufenden Nerv zu beschädigen, bei schlankeren Werkzeugen geringer.

Anhand der Figuren 1 bis 9 sei die Erfindung näher erläutert.

### Dabei zeigen

Fig. 1 und 1a schematisch eine PLIF- Versorgung,
Fig. 1b schematisch eine TLIF- Versorgung,
die Fig. 2 bis 2c verschiedene Ansichten eines erfindungsgemäßen Cages,
die Fig. 3, 4 und 5 Werkzeuge gemäß der Erfindung und
die Fig. 6, 7 und 8, wie durch entsprechende Gestaltung des Cage und der Werkzeuge diese geeignet sind, den Cage in verschiedene Positionen zu verbringen.

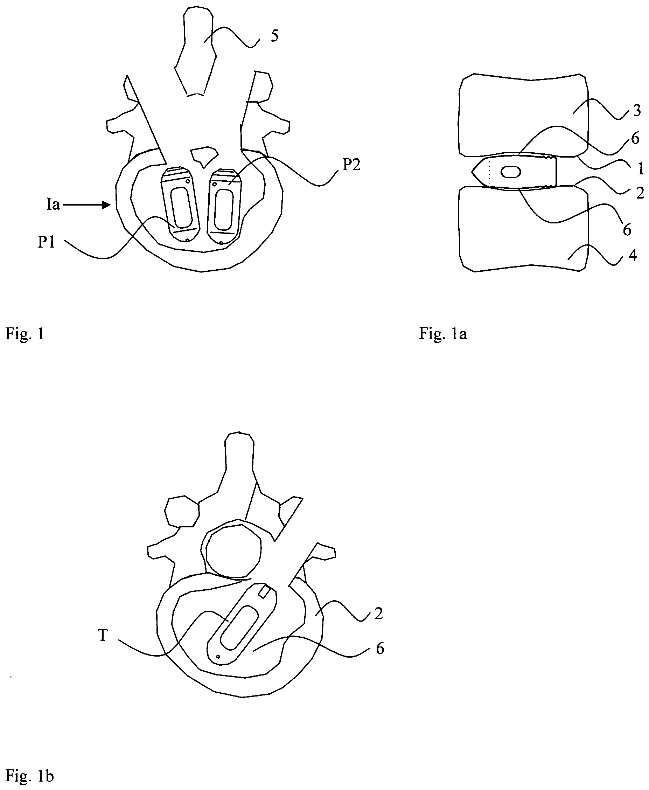

Die Figuren 1 und 1a zeigen schematisch eine Cage-Anordnung bei einer PLIF-Versorgung mit zwei Cages P1 und P2, die typischerweise 26mm lang sind, von dorsal eingebracht, jeweils in einer Position ca. 10° zur Körpermitte. Die Figur 1a zeigt dabei die beiden Cages innerhalb der Ringapophysen 1, 2 zweier benachbarter Wirbelkörper 3, 4. Der schematisch dargestellte Dornfortsatz ist in der Fig. 1 mit 5 bezeichnet. Die Figur 1 zeigt dabei die beiden Cages transversal und die Figur 1a eine Seitenansicht in einer Ansicht gemäß dem Pfeil Ia im Bandscheibenfach 6.

Figur 1b zeigt den bei der TLIF- Versorgung verwendeten einzigen Cage T im Bandscheibenfach 6 innerhalb Ringapophyse 2 eines Bandscheibenfaches in der für die TLIF typischen Lage, 30° bis 45° zur Körpermitte geneigt. In der Realität ist der Cage etwa 33mm lang.

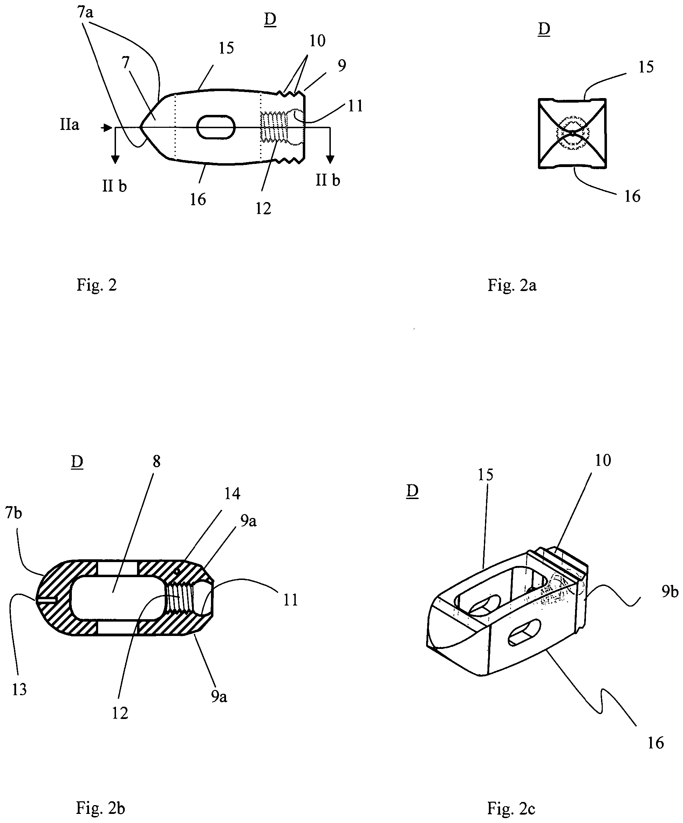

Figur 2 zeigt einen erfindungsgemäßen Cage D von der Seite her, Figur 2a den Cage D von vorne entsprechend dem Pfeil IIa. Die Figur 2b zeigt den Cage im Schnitt gemäß der Linie IIb-IIb und die Figur 2c den Cage gem. Fig. 2 aus einer Richtung von links schräg oben gesehen (aus der Sicht des Betrachters).

Der Cage hat eine längliche Struktur mit gerader Achse und ist an seiner Spitze 7 in der Seitenansicht entsprechend Fig.2 bei 7a facettenartig ausgebildet. In der Aufsicht entsprechend Fig. 2b ist die Spitze des Cages bei 7b abgerundet. Der Cage D ist käfigartig ausgebildet und besitzt hierfür eine durchgehende Ausnehmung 8. Diese Ausnehmung 8 ist bei diesem Cage zur Spitze hin versetzt; sie dient zur Aufnahme von Knochensplittern, die zuvor aus dem Zugangsbereich entnommen wurden, oder zur Aufnahme von synthetischem Knochenmaterial. Der hintere Endbereich 9 ist an der Ober- und Unterseite mit Riefen 10 versehen, die zur Fixierung und Sicherung gegen herausrutschen aus dem Bandscheibenfach dienen

Der Cage D weist an seinem hinteren Ende 9, von diesem Ende aus gesehen, zunächst eine kugelkalottenartige Profilierung 11 auf und - in Achsrichtung dahinter - eine Gewindebohrung 12.

Der Cage besitzt weiterhin an seinem vorderen Ende eine Ausnehmung 13 und an seinem hinteren Bereich eine Ausnehmung 14 zur Aufnahme von Markierungsstiften, die aus einem anderen Material bestehen als der Cage zur Sichtbarmachung der Lage des Cages während der Operation. Die Position des Markierungsstiftes 13 schließt präzise mit der Front des Cages ab und ist eine Besonderheit der vorliegenden Erfindung.

Der Cage kann zweckmäßigerweise aus Kunststoff hergestellt sein, z.B. aus PEEK, wobei es besonders vorteilhaft sein kann, wenn zumindest die in Richtung der aufeinander zu gerichteten Bandscheibenfächer weisenden Oberflächen 15, 16 aufgeraut sind, um einen besseren Halt zwischen den Wirbeln zu gewährleisten. Als besonders vorteilhaft hat sich das Quarzstrahlen der Oberflächen herausgestellt. Die Oberflächen können aber auch, gegebenenfalls zusätzlich, beschichtet sein, wobei sich eine Titanbeschichtung als besonders vorteilhaft erwiesen hat.

Der Cage entspricht in seiner Dimensionierung zumindest annähernd einem TLIF Cage.

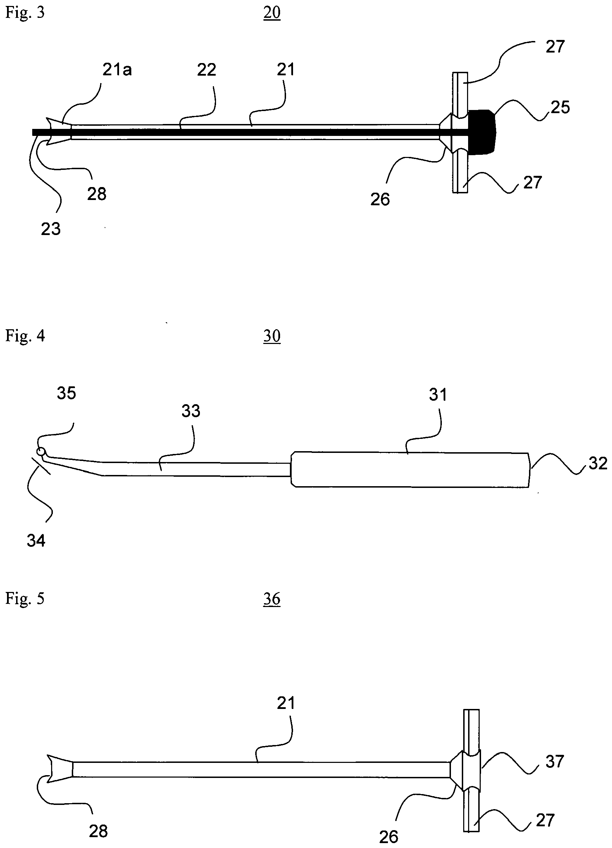

Figur 3 zeigt ein Werkzeug 20 zum Eintreiben eines Cages zwischen zwei Wirbel. Dieses Werkzeug - das Inserter Tool - besteht zunächst aus einem Hohlstab 21, in dem ein Innenteil oder Seele 22 vorgesehen ist. Diese Seele besitzt an ihrem distalen Ende einen Gewindebereich 23, der zur Aufnahme des Cages dient. Das proximale Ende der Seele ist fest mit dem Knopf bzw. Drehgriff 25 verbunden. Unterhalb des Knopfes 25 und am Hohlstab 21 mit dem Knauf 26 sind zwei Knebeln 27 vorgesehen. Das distale Ende 21a des Hohlstabes 21 ist gegenüber diesem erweitert und hat eine Auskehlung 28, die dem Heckbereich 9a (gem. Fig. 2b) zumindest teilweise angepasst ist, um dieses zumindest teilweise zu umgreifen.

Vor dem Einbringen des Cages in den Zwischenwirbelraum wird dieser mit seinem Gewindebereich 12 auf den Gewindebereich 23 der Seele aufgeschraubt und anschließend durch Verdrehen des Kopfes 25 gegenüber dem Knauf 26 mit seinen zwei Knebeln 27 der Cage fest gegen den fest mit dem Knauf verbundenen Hohlstab verspannt. Implantat D, Hohlstab 21, dessen am Implantat anliegender und an dessen Heckbereich beidseitig umgreifender, dem Heckbereich zumindest teilweise angepasster Endbereich 21a und Innenteil 22 sind dabei achsgleich zueinander angeordnet.

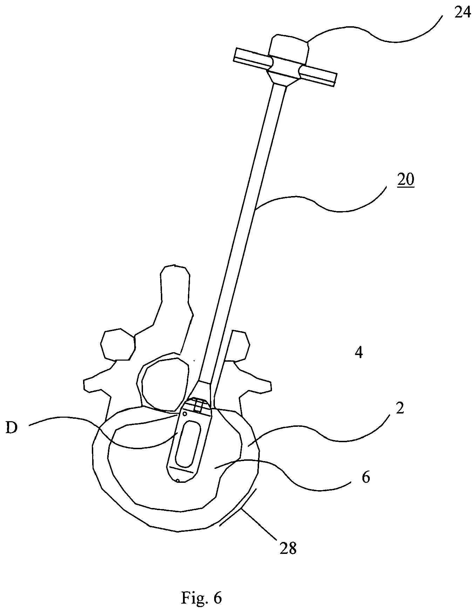

Figur 6 zeigt einen solchen Inserterstab 20 mit verschraubtem Cage D, teilweise eingebracht nach der PLIF- Methode durch Schläge auf den Kopf 24, in einem Winkel von etwa 10° zur Körpermitte, also in einer Winkellage, die dem PLIF entspricht. Der Cage D ist hier fast vollständig in den Bandscheibenraum 6 eingebracht und steht über den mit 28 angedeuteten Anulus und die Randleiste bzw. Ringapophyse 2 hinaus.

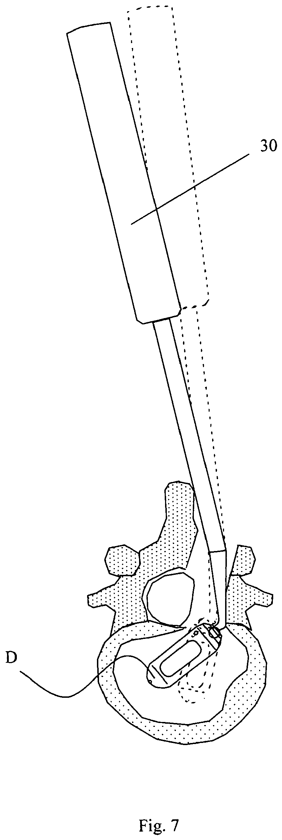

Um nun den Cage weiter in das Bandscheibenfach einzutreiben und auch in eine der TLIF- Lage zumindest weiter angenäherte Lage zu verbringen, sind Cage D und Werkzeug bzw. Ball Tool 30, welches in Figur 4 näher gezeigt ist, derart ausgestaltet, dass sie geeignet sind, den Cage aus der in Figur 7 punktiert eingezeichneten Position (die der Position der Figur 6 entspricht) in die stark ausgezeichnete Position verbracht zu werden, also in eine mehr diagonale Lage oder sogar noch über die TLIF-Lage hinaus.

Das Werkzeug 30 gemäß Fig. 4 weist an seinem proximalen Bereich einen Griff 31 auf und einen Beaufschlagungsbereich 32 für ein Schlagwerkzeug. Es besitzt einen stabartigen Bereich 33 und sein distaler Endbereich 35 ist gegenüber dem übrigen Bereich abgekröpft; er könnte auch bogenförmig verlaufen. Das distale Ende hat eine kugelige Kontur 35.

Nachdem aus der in Figur 6 entsprechenden Position das Werkzeug 20 durch Ausschrauben (Verdrehen des Knopfes 25 und damit der Seele 22) entfernt worden ist, kann nun das Werkzeug 30 mit seiner Kugel 35 in die Kalotte 11 (Fig 2, Fig 2b) des Cage D eingesetzt werden und durch Schläge auf den Bereich 32 (Fig-4) des Werkzeuges 30 wird der Cage D in die in Figur 7 stark ausgezeichnete Position verbracht. Beim Aufbringen von Schlagimpulsen auf den Griff des Werkzeuges 30 entstehen, bedingt durch die spezielle Formgebung, entstehen Kraftkomponenten, die den Cage sowohl weiter in den Bandscheibenraum verbringen als auch verschwenken; der Cage driftet gewissermaßen zwischen den benachbarten Ringhypophysen
hindurch bzw. an diesen entlang in eine, wie bereits erwähnt, zumindest annähernd der TLIF- Lage entsprechende Winkellage oder sogar darüber hinaus.

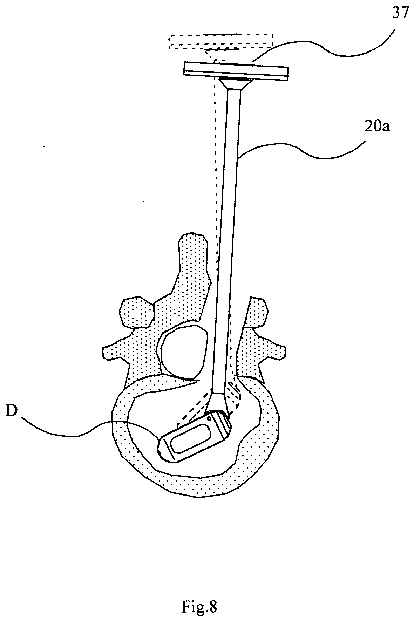

Für den Fall, dass die erreichte Position dem Operateur -dieser kann mittels der Markierungselemente die Lage des Cage feststellen -noch nicht optimal erscheint, kann mittels eines in Figur 5 dargestellten Werkzeuges 36 die Lage des Cage noch korrigiert werden. Das Werkzeug 36 entspricht hier - bis auf den mitsamt der Seele 22 entfernten Knopf 24 - dem in Fig. 3 dargestellten Werkzeug 20. Der Bereich 37 des Knaufes 26 dient als Schlagbereich für ein Werkzeug. Der distale Endbereich 28 dient zum Ansetzen des Werkzeuges am Heckbereich des Cage, wie dies in Figur 8 punktiert dargestellt ist, und durch Aufbringen von Schlägen auf den Schlagbereich 37 wird der Cage D noch weiter verschwenkt in die in Fig. 8 stark ausgezeichnete Position, die winkelmäßig auch über die TLIF- Lage hinausgehen kann, sogar in eine Position, in der Spitze und Heck des Cage zumindest annähernd auf gleicher Höhe liegen.

Wie insbesondere aus der Beschreibung und den Figuren hervorgeht, verwirklicht die vorliegende Erfindung letztlich auch ein Verfahren zum Implantieren von Cages nach der PLIF- Versorgung in die PLIF- Winkellage und das anschließende Verbringen des Cages in eine der TLIF- Versorgung zumindest annähernd entsprechende Position.

Die Erfindung bezieht sich darüberhinaus auf ein System zum Einbringen eines Wirbelsäulen-Implantates, das als länglicher Körper mit zumindest annähernd gerade verlaufender Achse ausgebildet ist, der auf wenigstens einem seiner Endbereiche eine erste Kontur als Angriffsfläche zur Bildung einer starren, jedoch lösbaren Verbindung mit dem distalen Endbereich eines ersten Werkzeuges aufweist, geeignet zum teilweisen, vorzugsweise jedoch überwiegenden Einbringen zwischen zwei benachbarten Wirbeln einer Wirbelsäule nach dem (Posterior Lumbar Interbody Fusion) PLIF- Verfahren, vorzugsweise mit seinem hinteren Ende noch zwischen den Ringapophysen der beteiligten Wirbelkörper befindlichen Lage durch Aufbringen von Schlagimpulsen auf den proximalen Endbereich des Werkzeuges, wobei das Implantat eine weitere Kontur als Angriffsfläche zur Bildung einer gelenkigen Verbindung mit einem Werkzeug aufweist, geeignet zum Verbringen in eine schräge, dem TLIF- Verfahren zumindest annähernd entsprechende Lage und wobei die erste und die weitere Kontur innerhalb des Implantats vorgesehen sind. Das System umfasst ein weiteres Werkzeug, geeignet -oder eines der vorhandenen Werkzeuge ist umrüstbar und geeignet -, um nach Entfernen des zweiten Werkzeuges, durch die Präparation hindurch, mit einem gabel- bzw. zinkenartigen Bereich an dem Heckbereich des Implantats angesetzt zu werden zur weiteren Verlagerung desselben mittels auf das proximale Ende des Werkzeuges aufzubringender Schlagimpulse.

## Patentansprüche

1. Wirbelsäulen-Implantat, das als länglicher Körper mit praktisch gerade verlaufender Achse ausgebildet ist, der auf wenigstens einem seiner Endbereiche eine Kontur als Angriffsfläche zur starren, jedoch lösbaren Verbindung mit einem ersten Werkzeug und eine weitere Kontur als Angriffsfläche zur gelenkigen Verbindung mit einem anderen Werkzeug aufweist und beide Konturen innerhalb des Implantats, praktisch achsgleich zueinander und auf der über die Länge des Implantats gerade verlaufenden Achse desselben liegend, vorgesehen sind.

2. Wirbelsäulen-Implantat nach Anspruch 1, **dadurch gekennzeichnet, dass** die Kontur der gelenkigen Verbindung hohlkörperartig, insbesondere kalottenartig, vorzugsweise kugelkalottenartig ausgebildet ist.

3. Wirbelsäulen-Implantat nach einem der Ansprüche 1 oder 2, **dadurch gekennzeichnet, dass** die Konturen ineinander übergehen.

4. Wirbelsäulen-Implantat nach einem der Ansprüche 1 bis 3, **dadurch gekennzeichnet, dass** der Bug des Cage bombiert ist.

5. Wirbelsäulen-Implantat nach einem der Ansprüche 1 bis 4, **dadurch gekennzeichnet, dass** das Heck des Cage an den bandscheibenfach-seitig zu liegen kommenden Oberflächen profiliert ist.

6. Wirbelsäulen-Implantat nach einem der Ansprüche 1 bis 5, **dadurch gekennzeichnet, dass** es eine dritte Kontur für den Angriff eines Werkzeuges mit gabelförmig ausgebildetem distalen Ende aufweist.

7. Wirbelsäulen-Implantat nach einem der Ansprüche 1 bis 6, **dadurch gekennzeichnet, dass** die dritte Kontur eine der gelenkartigen Angriffsfläche benachbarte Kontur des Implantats ist.

8. Wirbelsäulen-Implantat nach einem der Ansprüche 1 bis 7, **dadurch gekennzeichnet, dass** die dritte Kontur eine der Außenkonturen des Implantats ist.

9. Wirbelsäulen-Implantat nach einem der vorhergehenden Ansprüche, das eine längliche Struktur mit praktisch gerader Achse, an zumindest einem seiner Endbereiche einen eingebrachten kalottenartigen oder zylinderförmigen Hohlkörper, insbesondere eine kugelkalottenartige Einbuchtung und weiter innen, praktisch achsgleich mit dieser eine Gewindebohrung aufweist, wobei diese und die Einbuchtung auf der durchgehend praktisch geraden Achse des Implantats liegen.

10. Werkzeug zum Implantieren eines Wirbelsäulen-Implantats, insbesondere nach einem der vorhergehenden Ansprüche mit einem Hohlstab, der an seinem proximalen Ende einen ebenfalls hohlen Griff aufweist zur Aufnahme eines Innenstabes, der mit einem über das distale Ende des Hohlstabes hinausreichenden, am Innenstab einstückig und praktisch achsgleich angeformten Gewindebereich versehen ist zur Aufnahme und zur Verspannung des Implantates mit dessen Heckbereich gegen den distalen Endbereich des Hohlstabes, der dem Heckbereich des Implantats zumindest teilweise angepasst ist an diesem beidseits des Innenstabes zur Anlage bringbar ist und wobei zweckmäßigerweise Innenstab, Hohlstab, distaler Endbereich desselben und das praktisch gerade ausgebildete Implantat praktisch annähernd achsgleich zueinander fest verbindbar sind.

11. Werkzeug zum Implantieren eines Wirbelsäulen-Implantats nach Anspruch 10, **gekennzeichnet durch** eine stabförmige Ausbildung mit einem proximalen Endbereich als Handgriff und einem distalen Endbereich, wobei die Endbereiche versetzt, insbesondere achsversetzt zueinander vorgesehen sind und dass das distale Ende ballig, wie kugelförmig ausgebildet ist, geeignet, um in eine hohlkörperartige Kontur innerhalb des Implantats eingesetzt zu werden und damit eine gelenkige Verbindung zu schaffen, um durch Aufbringen von Schlägen auf den Handgriff das Implantat innerhalb zweier benachbarter Wirbel zu verlagern.

12. Werkzeug zum Implantieren eines Wirbelsäulen-Implantats nach Anspruch 11, **dadurch gekennzeichnet, dass** sein distaler Bereich abgekröpft ist.

13. System zum Einbringen eines Wirbelsäulen-Implantates, das als länglicher Körper mit zumindest annähernd gerade verlaufender Achse ausgebildet ist, der auf wenigstens einem seiner Endbereiche eine erste Kontur als Angriffsfläche zur Bildung einer starren, jedoch lösbaren Verbindung mit dem distalen Endbereich eines ersten Werkzeuges aufweist, geeignet zum teilweisen, vorzugsweise jedoch überwiegenden Einbringen zwischen zwei benachbarten Wirbeln einer Wirbelsäule nach dem Posterior Lumbar Interbody Fusion - Verfahren, vorzugsweise mit seinem hinteren Ende noch zwischen den Ringapophysen der beteiligten Wirbelkörper befindlichen Lage durch Aufbringen von Schlagimpulsen auf den proximalen Endbereich des Werkzeuges, wobei das Implantat eine weitere Kontur als Angriffsfläche zur Bildung einer gelenkigen Verbindung mit einem Werkzeug aufweist, geeignet zum Verbringen in eine schräge, dem TLIF- Verfahren zumindest annähernd entsprechende Lage und wobei die erste und die weitere Kontur innerhalb des Implantats vorgesehen sind.

14. System nach Anspruch 13, **dadurch gekennzeichnet, dass** die erste, gegenüber der weiteren Kontur weiter innen liegende Kontur ein Innengewinde ist, vorgesehen zur starren Aufnahme eines an seinem distalen Ende mit einem Gewindezapfen versehenen ersten Werkzeuges und zum lediglich teilweisen Einbringen des Implantats zwischen zwei benachbarte Wirbel und wobei die weitere Kontur kalottenartig, insbesondere kugelkalottenartig oder in der Form eines walzenförmigen Hohnzylinders ausgebildet und geeignet ist zur vorübergehenden Aufnahme eines am distalen Endbereich eines weiteren Werkzeuges vorgesehenen Gegenprofiles, insbesondere eines kugelartig ausgebildeten Bereiches zur Verbringung in eine zumindest annähernd der TLIF- Versorgung entsprechende Lage.

15. System nach Anspruch 13 oder 14, **dadurch gekennzeichnet, dass** der distale Endbereich des weiteren Werkzeuges gegenüber dem proximalen Endbereich versetzt ist, geeignet, um beim Aufbringen von Schlagimpulsen auf den letzteren, das Implantat in eine zumindest annähernd diagonale Lage zu driften.

16. System nach einem der Ansprüche 13 bis 15, **dadurch gekennzeichnet, dass** das Implantat eine zusätzliche Kontur aufweist zum Ansetzen eines zinken- oder gabelartig ausgebildeten distalen Endbereiches eines zusätzlichen Werkzeuges, geeignet zur weiteren Verlagerung des Implantates beim Aufbringen von Schlagimpulsen auf das proximale Ende des weiteren Werkzeuges.

17. System nach Anspruch 16, **dadurch gekennzeichnet, dass** die zusätzliche Kontur eine der Endkonturen des Implantats ist, insbesondere die zumindest annähernd senkrecht zur Transversalebene des Bandscheibenfaches verlaufende Begrenzungskontur, insbesondere eine der ersten Kontur benachbarte Begrenzungskontur des Implantates.

## Claims

1. Spinal implant, which is designed as an elongated body with a practically straight axis, which on at least one of its end parts has a contour as a contact surface for forming a rigid, but detachable connection with a first tool and an additional contour as a contact surface for forming a joint connection with another tool and both contours are provided inside the implant, are practically aligned with one another and are on the axis running in a straight line over the length of the implant.

2. Spinal implant according to claim 1, **characterised in that** the contour of the joint connection is designed in the form of a hollow body, in particular as a spherical cap, preferably as a ball-like spherical cap.

3. Spinal implant according to either of claims 1 or 2, **characterised in that** the contours merge into one another.

4. Spinal implant according to any of claims 1 to 3, **characterised in that** the nose of cage is curved.

5. Spinal implant according to any of claims 1 to 4, **characterised in that** the rear of the cage is profiled on the surfaces which come to bear on the side of the intervertebral disc space.

6. Spinal implant according to any of claims 1 to 5, **characterised in that** it has a third contour for the engagement of a tool with a fork-like distal end.

7. Spinal implant according to any of claims 1 to 6, **characterised in that** the third contour is a contour of the implant adjacent to the joint-like contact surface.

8. Spinal implant according to any of claims 1 to 7, **characterised in that** the third contour is one of the external contours of the implant.

9. Spinal implant according to any of the preceding claims, which has an elongated structure with a practically straight axis, on at least one of its end parts has an inserted spherical cap-like or cylindrical hollow body, in particular a ball-like, spherical cap-shaped indentation and further inside has a threaded bore practically aligned with the latter, wherein the threaded bore and the indentation are on the continuous practically straight axis of the implant.

10. Tool for implanting a spinal implant, in particular according to any of the preceding claims with a hollow rod, which at its proximal end has an also hollow handle for receiving an inner rod, which is provided with a threaded area extending over the distal end of the hollow rod and formed in one piece on the inner rod and in practically the same alignment for mounting and tensioning the implant with its rear part against the distal end part of the hollow rod, which is adjusted at least partly to the rear part of the implant and can be moved into position on the latter on both sides of the inner rod and wherein preferably the inner rod, hollow rod, distal end part thereof and the practically straight implant can be connected securely to one another at least approximately with the same alignment.

11. Tool for implanting a spinal implant according to claim 10, **characterised by** a rod-like design with a proximal end part as a handle and a distal end part, wherein the end parts are offset, in particular are offset axially to one another, and the distal end is designed to be spherical or ball-like, suitable for being inserted into a hollow body-like contour inside the implant and thus forming a joint connection in order to move the implant within two adjacent vertebrae by tapping the handle.

12. Tool for implanting a spinal implant according to claim 11, **characterised in that** its distal part is bent.

13. System for inserting a spinal implant, which is designed as an elongated body with an at least approximately straight axis, which on at least one of its end parts has a first contour as an contact surface for forming a rigid, but detachable connection with the distal end part of a first tool, suitable for partly but preferably mostly inserting between two adjacent vertebrae of a spinal column according to the Posterior Lumbar Interbody Fusion method, preferably with its rear end still between the ring apophyses of the affected vertebral bodies by applying tapping pulses to the proximal end part of the tool, wherein the implant has an additional contour as a contact surface for forming a joint connection with a tool, suitable for moving into an oblique position corresponding at least approximately to the TLIF method and wherein the first and the additional contour are provided inside the implant.

14. System according to claim 13, **characterised in that** the first contour further inside than the additional contour is an internal thread, provided for rigidly mounting a first tool provided at its distal end with a threaded pin and for only partly inserting the implant between two adjacent vertebrae and wherein the additional contour is designed to be like a spherical cap, in particular a ball-like spherical cap or in the form of a roller-like hollow cylinder and is suitable for temporarily mounting a counter profile provided on the distal end part of an additional tool, in particular a ball-like part for moving into a position corresponding at least approximately to the TLIF technique.

15. System according to claim 13 or 14, **characterised in that** the distal end part of the additional tool is offset relative to the proximal end part, to be suitable, when applying tapping pulses onto the latter, for drifting the implant into an at least approximately diagonal position.

16. System according to any of claims 13 to 15, **characterised in that** the implant has an additional contour for attaching a prong-like or fork-like distal end part of an additional tool, suitable for the additional displacement of the implant when applying tapping pulses to the proximal end of the additional tool.

17. System according to claim 16, **characterised in that** the additional contour is one of the end contours of the implant, in particular the delimiting contour running approximately perpendicular to the transverse plane of the intervertebral disc space, in particular a delimiting contour of the implant adjacent to the first contour.

## Revendications

1. Implant de colonne vertébrale qui est réalisé sous forme de corps allongé avec un axe s'étendant pratiquement rectiligne, lequel présente sur au moins une de ses zones d'extrémité un contour servant de surface d'attaque pour réaliser une liaison rigide mais amovible avec un premier outil et un autre contour servant de surface d'attaque pour réaliser une liaison articulée avec un autre outil, et les deux contours étant prévus à l'intérieur de l'implant, pratiquement coaxiaux l'un par rapport à l'autre et situés sur l'axe de l'implant s'étendant rectiligne sur la longueur de celui-ci.

2. Implant de colonne vertébrale selon la revendication 1, **caractérisé en ce que** le contour de la liaison articulée est réalisé en forme de corps creux, en particulier en forme de calotte, de préférence en forme de calotte sphérique.

3. Implant de colonne vertébrale selon l'une des revendications 1 ou 2, **caractérisé en ce que** les contours passent l'un dans l'autre.

4. Implant de colonne vertébrale selon l'une des revendications 1 à 3, **caractérisé en ce que** la partie avant de la cage est bombée.

5. Implant de colonne vertébrale selon l'une des revendications 1 à 4, **caractérisé en ce que** la partie arrière de la cage est profilée sur les surfaces qui viennent se placer sur le côté du disque intervertébral.

6. Implant de colonne vertébrale selon l'une des revendications 1 à 5, **caractérisé en ce qu'**il présente un troisième contour pour l'attaque d'un outil avec une extrémité distale réalisée en forme de fourchette.

7. Implant de colonne vertébrale selon l'une des revendications 1 à 6, **caractérisé en ce que** le troisième contour est un contour de l'implant qui est voisin de la surface d'attaque en forme d'articulation.

8. Implant de colonne vertébrale selon l'une des revendications 1 à 7, **caractérisé en ce que** le troisième contour est un des contours extérieurs de l'implant.

9. Implant de colonne vertébrale selon l'une des revendications précédentes, qui présente une structure allongée avec axe pratiquement rectiligne, un corps creux en forme de calotte ou de cylindre introduit à au moins une de ses zones d'extrémité, en particulier une échancrure en forme de calotte sphérique et, plus vers l'intérieur, pratiquement coaxialement avec celle-ci, un perçage fileté, celui-ci et l'échancrure se trouvant sur l'axe pratiquement entièrement droit de l'implant.

10. Outil permettant d'implanter un implant de colonne vertébrale, en particulier selon l'une des revendications précédentes, comportant une tige creuse qui présente à son extrémité proximale une poignée également creuse destinée à recevoir une tige intérieure, qui est pourvue d'une section filetée formée d'un seul tenant et pratiquement coaxialement sur la tige intérieure et dépassant l'extrémité distale de la tige creuse, pour recevoir et serrer l'implant avec sa zone arrière contre la zone d'extrémité distale de la tige creuse, qui est adaptée au moins partiellement à la zone arrière de l'implant et qui peut être mise en contact sur celle-ci des deux côtés de la tige intérieure, et de manière appropriée, la tige intérieure, la tige creuse, la zone d'extrémité distale de celle-ci et l'implant réalisé pratiquement rectiligne pouvant être solidarisé(e)s les un(e)s aux autres pratiquement de manière approximativement coaxiale.

11. Outil permettant d'implanter un implant de colonne vertébrale selon la revendication 10, **caractérisé par** une réalisation en forme de tige avec une zone d'extrémité proximale servant de poignée et une zone d'extrémité distale, les zones d'extrémité étant prévues en décalage l'une par rapport à l'autre, en particulier en décalage axial, et en ce que l'extrémité distale est réalisée bombée, telle que de forme sphérique, et est appropriée pour être introduite dans un contour en forme de corps creux à l'intérieur de l'implant et pour créer ainsi une liaison articulée pour déplacer l'implant à l'intérieur de deux vertèbres voisines en frappant sur la poignée.

12. Outil permettant d'implanter un implant de colonne vertébrale selon la revendication 11, **caractérisé en ce que** sa zone distale est coudée.

13. Système permettant d'introduire un implant de colonne vertébrale qui est réalisé sous forme de corps allongé avec un axe s'étendant au moins approximativement rectiligne, lequel présente sur au moins une de ses zones d'extrémité un premier contour servant de surface d'attaque pour former une liaison rigide mais amovible avec la zone d'extrémité distale d'un premier outil, qui est approprié pour introduire partiellement, de préférence toutefois essentiellement, l'implant entre deux vertèbres voisines d'une colonne vertébrale selon le procédé dit Posterior Lumbar Interbody Fusion, de préférence avec son extrémité postérieure encore dans une position se trouvant entre les apophyses annulaires des corps des vertèbres concernées, en appliquant des impulsions de frappe sur la zone d'extrémité proximale de l'outil, l'implant présentant un autre contour servant de surface d'attaque pour former une liaison articulée avec un outil, qui est approprié pour l'amener dans une position oblique correspondant au moins approximativement au procédé dit TLIF, et le premier et l'autre contour étant prévus à l'intérieur de l'implant.

14. Système selon la revendication 13, **caractérisé en ce que** le premier contour situé plus à l'intérieur par rapport à l'autre contour est un filetage intérieur prévu pour recevoir de manière rigide un premier outil muni d'un embout fileté à son extrémité distale et pour introduire seulement partiellement l'implant entre deux vertèbres voisines, et l'autre contour étant réalisé en forme de calotte, en particulier en forme de calotte sphérique ou sous la forme d'un cylindre creux en forme de rouleau et étant approprié pour recevoir provisoirement un profil antagoniste prévu sur la zone d'extrémité distale d'un autre outil, en particulier d'une zone réalisée en forme de sphère pour être amené dans une position correspondant au moins approximativement aux traitements selon le TLIF.

15. Système selon la revendication 13 ou 14, **caractérisé en ce que** la zone d'extrémité distale de l'autre outil est décalée par rapport à la zone d'extrémité proximale et appropriée, lors de l'application d'impulsions de frappe sur cette dernière, pour dériver l'implant dans une position au moins approximativement diagonale.

16. Système selon l'une des revendications 13 à 15, **caractérisé en ce que** l'implant présente un contour additionnel pour placer une zone d'extrémité distale réalisée en forme de dents ou de fourchette, d'un outil additionnel, approprié pour déplacer encore l'implant lors de l'application d'impulsions de frappe sur l'extrémité proximale de l'autre outil.

17. Système selon la revendication 16, **caractérisé en ce que** le contour additionnel est un des contours d'extrémité de l'implant, en particulier le contour de limitation s'étendant au moins approximativement perpendiculairement au plan transversal du disque intervertébral, en particulier un contour de limitation de l'implant qui est voisin du premier contour.
